# EUROPÄISCHE PATENTANMELDUNG

(11) **EP 4 541 311 A2**
(43) Veröffentlichungstag der Anmeldung: **23.04.2025**
(21) Anmeldenummer: 25161450.9
(22) Anmeldetag: 10.08.2021
(51) Int. Cl.: A61C 13/271

(54) **DENTALER ROHLING UND HERSTELLUNG EINE DENTALEN TEILPROTHESE AUS EINEM DENTALEN ROHLING**

(62) Teilanmeldung aus: 21190668.0
(71) Anmelder: Ivoclar Vivadent AG, 9494 Schaan (LI)
(72) Erfinder: Watzke, Ronny, 6800 Feldkirch (AT)
(74) Vertreter: Baldus, Oliver

(57) **Zusammenfassung**

Die vorliegende Erfindung betrifft einen dentalen Rohling, insbesondere einen quaderförmigen Block, mit einer oberen und einer unteren Fläche, der aus einem fleischfarbenen Material und einem zahnfarbenen Material, aufgebaut ist. Das fleischfarbene Material und das zahnfarbene Material sind miteinander verbunden. Die Grenzfläche zwischen den Materialien mit in oder an der Grenzfläche ausgebildeten Erhöhungen und Vertiefungen erstreckt sich durch eine gegebenenfalls gebogene Ebene, welche Ebene parallel zu oder schräg zu mindestens einem Teil der Flächen des Rohlings liegt. Im Rohling ist ein Bereich für mindestens eine Teilprothese reserviert, der sich über eine oder mehrere Erhöhungen und Vertiefungen erstreckt und die Scheitellinien der Erhöhungen und Vertiefungen in der Draufsicht auf die Grenzfläche betrachtet im Wesentlichen gerade Linien ausbilden, welche über den Block betrachtet fächerförmig angeordnet sind oder voneinander divergieren.

## Beschreibung

Die Erfindung betrifft einen Block mit einer oberen und einer unteren Fläche gemäß dem Oberbegriff von Anspruch 1, einen dentalen Rohling mit einer oberen und einer unteren Fläche gemäß dem Oberbegriff von Anspruch 5, eine dentale Teilprothese gemäß dem Oberbegriff von Anspruch 7 und ein Verfahren zur Herstellung einer dentalen Teilprothese gemäß dem Oberbegriff von Anspruch 8.

Es ist seit langem bekannt, Prothesen, aber auch Zähne, aus mehreren Schichten aufzubauen. Als Beispiele hierfür seien die aus der WO 90/13268 A1 und aus der WO 91/07141 A1 bekannten Lösungen genannt.

Für Dentalprothesen ist zum einen eine gute Materialverträglichkeit und zum anderen eine schlanke Gestaltung der Prothese oder Teilprothese wünschenswert. Um eine sichere Verankerung der Zähne in der Dentalprothese zu gewährleisten, werden diese typischerweise geklebt oder aber auch in einem Spritzgussverfahren eingebracht. Eine diesbezügliche Lösung ist aus der DE 837 288 B1 ersichtlich.

Eine Teilprothese muss weiterhin eine sichere Verankerung im Kiefer des Patienten ermöglichen. Hierzu werden häufig Metallgerüste eingebettet, welche eine Fixierung an den Nachbarzähnen oder an Implantaten im Kiefer des Patienten erlauben.

Derzeit werden für die Herstellung von derartigen Teilprothesen mehrere Verfahrensschritte benötigt. Zunächst muss ein Gerüst aus Metall oder gegebenenfalls aus Kunststoff erstellt werden, welches im Anschluss in ein Wachs-Modell eingebettet wird. Anschließend wird dieses Wachs-Modell, beispielsweise in einem Gießverfahren oder über das Lost-Wax-Verfahren, durch das Prothesenmaterial ersetzt. Im Anschluss wird die Teilprothese final ausgearbeitet und poliert. Ein derartiges mehrschrittiges Verfahren ist beispielsweise aus der WO 2007/060142 A1 bekannt.

Wie beschrieben, bestehen Teilprothesen aus mehreren Teilen und unterschiedlichen Materialien. Die Grösse von Teilprothesen richtet sich nach der jeweiligen Zahnsituation eines Patienten. Dies kann im Vergleich zu einer Vollprothese individuell sehr unterschiedlich sein, da jeder Patient eine unterschiedliche Anzahl und Ausdehnung von Lücken hat.

Da das Modellieren der Gingiva- und Zahnanteile in Wachs sowie das Überführen in die finale Prothese fehleranfällig und aufwendig ist, kann das Arbeiten mit mehreren Modellen sehr leicht zu Transferfehlern führen. Die Fertigung von Teilprothesen ist daher zeitintensiv und aufgrund der Vielzahl an Materialien und Arbeitsschritten sehr fehleranfällig.

Daher liegt der Erfindung die Aufgabe zugrunde, einen Block mit einer oberen und einer unteren Fläche gemäß dem Oberbegriff von Anspruch 1, einen dentalen Rohling mit einer oberen und einer unteren Fläche gemäß dem Oberbegriff von Anspruch 5, eine dentale Teilprothese gemäß dem Oberbegriff von Anspruch 7 und ein Verfahren zur Herstellung einer dentalen Teilprothese gemäß den Oberbegriff von Anspruch 8 zu schaffen, die zum einen preisgünstig und schnell zu fertigen ist und zum anderen eine geringere Fehleranfälligkeit aufweist und auch hinsichtlich der Lagerhaltungsmöglichkeiten optimiert ist.

Diese Aufgabe wird erfindungsgemäß durch einen Gegenstand nach Anspruch 1, 5, 7 bzw. 8 gelöst. Vorteilhafte Weiterbildungen ergeben sich aus den Unteransprüchen.

Erfindungsgemäß ist es vorgesehen, dass die Teilprothese aus einem besonders gestalteten Rohling gefertigt wird. Der Rohling ist zweifarbig gestaltet und besteht aus einem Zahnfleisch-farbenen (rosa bis rötlichen) Material und einem Zahn-farbenen (weißlich bis beigen) Material, insbesondere je auf Kunststoffbasis oder aus einem keramischen Material, welche in einer erfindungsgemäßen Form miteinander verbunden sind. Diese intensive Verbindung kann beispielsweise durch Kleben oder aber auch durch Polymerisieren erreicht werden. Auch ist es möglich, dass die beiden Materialien nur mittels einer Pressvorrichtung, wie beispielsweise einer oder mehrerer Schraubzwingen, zusammengehalten werden, aber jederzeit separiert werden können.

Die einteilige und untrennbare Herstellung kann beispielsweise dadurch erzielt werden, dass die Materialien in einem an der Grenzfläche noch weichen - oder gar flüssigen - Zustand gegen einander gepresst werden, so dass sie - mikroskopisch betrachtet - ineinander eindringen. Der Übergangsbereich ist aber auch bei dieser Art der Herstellung im Submillimeterbereich, beispielsweise weniger als 100 µm dick.

Auch beim Aufpolymerisieren besteht ein derartiger Übergangsbereich in der gleichen Größenordnung, und beim Kleben kann die Klebefuge eine geringe Stärke zwischen beispielsweise 40 und 200 µm aufweisen.

Die Materialien bestehen bevorzugt aus einem Polymer, insbesondere PMMA, jedoch sind auch andere Materialien wie beispielsweise Keramiken, insbesondere Zirkondioxid, oder Metalle denkbar. Auch ist es möglich, zwei unterschiedliche Materialien zu verwenden.

Erfindungsgemäß ist der Rohling als zweifarbiger Block oder als zweifarbige Scheibe oder als im wesentlichen scheibenförmiger, insbesondere flachzylindrischer, Rohling mit einer oberen und einer unteren Fläche gestaltet. Unter Scheibe sei z.B. ein Block mit einer flachen Form zu verstehen, die insbesondere abgerundet ist, oder sogar rund. Eine derartige zweifarbige Scheibe kann beispielsweise ein (Flach-) Zylinder sein, bei dem die Höhe um vieles geringer ist als der Radius. Diese Höhe des Zylinders kann sich in der Drehachse des Zylinders erstrecken und würde in der Schoenflies-Symbolik mit C∞ bezeichnet, wobei "C" zyklisch bedeutet und der Index die Zähligkeit, also hier ∞ für die Rotationssymmetrie, angibt. Die Ebene, die die Grenzfläche zwischen den beiden Materialien durchtritt, verläuft senkrecht zur Höhe, also senkrecht zur Rotationsachse des Zylinders und entspricht daher einer Rotationsfläche oder Drehfläche.

In vorteilhafter Ausführung ist der Rohling ein Block oder ein scheibenförmiger Körper. Dieser kann unrund, insbesondere mehreckig, aber auch rund gestaltet sein. Eine mehreckige Form lässt sich beispielsweise durch einen flachen Quader oder geometrisch hierzu ähnlichen Körper realisieren, bei dem eine der Seiten kürzer als die anderen beiden ist. Jedoch sind auch andere, insbesondere flache, Polyeder wie ein einheitlich gleichseitiges achteckiges Antiprisma oder ein einheitlich gleichseitiges, z.B. dekagonales Antiprisma möglich, ohne den Bereich der Erfindung zu verlassen.

Die Grenzfläche zwischen den Materialien des Rohlings verläuft entlang der Ebene, muss aber nicht flach bzw. eben ausgestaltet sein. Die Ebene, in welcher die Grenzfläche liegt, ist in einer bevorzugten Ausgestaltung parallel zu einer der ggf. ebenen oberen bzw. unteren Flächen des Rohlings oder Blocks angeordnet, insbesondere zu einer derjenigen, die den größten Flächeninhalt hat. Diese Ebene kann aber auch diagonal im Rohling liegen. Wenn die Flächen nicht eben sind, sondern z.B. gewölbt oder strukturiert, kann sich die Ebene im wesentlichen parallel oder parallel zu einem Teil der Fläche erstrecken. Unter «im wesentlichen parallel» ist hier und im übrigen eine Erstreckung mit einer geringen Winkelabweichung zur Parallelität zu verstehen, mit bevorzugt weniger als 20 Grad, besonders bevorzugt weniger als 10 Grad und insbesondere weniger als 5 Grad.

In einer weiteren Ausgestaltung ist es vorgesehen, dass die Ebene der Grenzfläche zwischen den Materialien des Rohlings selbst nicht eben ist, sondern eine Wölbung aufweist. Die Grenzfläche zwischen den Materialien des Rohlings kann in diesem Fall mit einem Gewölbe, einem Kegel oder einer Pyramide vergleichen werden. Insbesondere ist ein Verlauf entsprechend der Spee-Kurve bevorzugt.

Das fleischfarbene Material weist an der Grenzfläche zwischen den beiden Materialien Erhöhungen und Vertiefungen auf, welche jeweils aus der Ebene hinausragen. Die Scheitel einer Vertiefung oder einer Erhöhung bilden je eine Linie, insbesondere eine gerade Linie, welche parallel oder zumindest annährend parallel zur Grenzfläche verläuft. Weiterhin verlaufen die einzelnen Scheitellinien der Erhöhungen und Vertiefungen jeweils im Wesentlichen parallel zueinander.

Jede der Teilprothesen wird bevorzugt aus einem Bereich des Rohlings herausgearbeitet, insbesondere herausgefräst, der sich über eine oder mehrere Erhöhungen und Vertiefungen erstreckt.

In einer weiteren Ausgestaltung sind die Scheitellinien geschwungen oder auch gezackt oder weisen gerade Bereiche sowie auch geschwungene oder gezackte Bereiche auf.

Die Linien weisen bevorzugt einen konstanten Abstand zueinander auf, jedoch ist es auch möglich, dass der Abstand zwischen benachbarten Scheitellinien unterschiedlich ist. Beispielsweise ist es möglich, dass der Abstand der Scheitellinien, welche näher am Mittelpunkt der Grenzfläche liegen, abnimmt und nach Außen hin, also in Richtung Rand des Rohlings, zunimmt.

Vorteilhaft sind jedoch auch unregelmässige Anordnungen der Abstände, also Bereiche des Rohlings, welche breite Abstände zwischen den Scheitellinien aufweisen sowie Bereiche des Rohlings, welche sehr enge Abstände zwischen den Scheitellinien zeigen.

In einer weiteren Ausgestaltung sind die im Wesentlichen geraden Scheitellinien nicht parallel, sondern fächerförmig angeordnet, so dass die Abstände zwischen den Scheitellinien an einer Seite des Rohlings enger und an der gegenüberliegenden Seite des Rohlings breiter ausgestaltet sind.

Die Form der Teilprothese selbst kann in beliebiger geeigneter Weise gewählt werden. Die Erstreckung der Teilprothese ist bevorzugt über mehrere Linien hinweg, jedoch ist auch eine Anordnung über nur eine Scheitellinie, genauer eine Scheitellinie einer Vertiefung des fleischfarbenen Materials, hinweg möglich.

Bevorzugt weist das zahnfarbene Material an der Grenzfläche zwischen den beiden Materialien exakt die Negativform zum fleischfarbenen Material auf. Somit weist die Grenzfläche zwischen den Materialien Erhöhungen und Vertiefungen auf, so dass die beiden Materialien ineinander greifen und gleichsam miteinander verzahnt sind.

In vorteilhafter Ausgestaltung kann das fleischfarbene Material auf das zahnfarbene Material aufpolymerisiert werden. In einer weiteren Ausführungsform befindet sich - wie schon oben beschrieben - zwischen den beiden Materialien eine Schicht aus Klebemasse, Füge- oder Verbundmaterial, um die beiden Materialien fest zu verbinden. Auch ist es ggf. besonders günstig, die beiden Materialien über additive Verfahren oder durch Aufpressen oder andere Verbindungstechniken zu verbinden.

Betrachtet man den Rohling im Querschnitt durch beide Materialien, also senkrecht zur Ebene, wobei hier angenommen wird, dass das fleischfarbene Material unten und das zahnfarbene Material oben ist und welcher Querschnitt bevorzugt durch den Mittelpunkt des Rohlings und senkrecht zu den Linien verläuft, so zeigt das fleischfarbene Material an der Grenzfläche Erhöhungen und Vertiefungen, welche bevorzugt nicht symmetrisch sind. Die Erhöhungen des fleischfarbenen Materials sind eher spitz zulaufend gestaltet, während die Vertiefungen des fleischfarbenen Materials eher ausgerundet sind. Insofern liegen - in der Seitenansicht betrachtet - Scheitel vor, und sich von diesen nach unten wegerstreckende Schrägflächen, die Hänge bilden und in Tälern auslaufen. Diese Gestaltung gleicht einer Aneinanderreihung von Us oder einer Kettenlinie. Diese erfindungsgemäße Gestaltung der Grenzfläche des Blocks, Rohlings oder der teilbearbeiteten Prothese erlaubt es auf eine einfache Weise die Form und den Verlauf der Zähne und/der des Zahnfleisches für die finale Prothese so festzulegen, dass ohne großen Aufwand beim Herstellungsvorgang der finalen Prothese ein natürlicher menschlicher Zahnfleischsaum dargestellt werden kann.

Das zahnfarbene Material zeigt in diesem Querschnitt an der Grenzfläche zwischen den beiden Materialien exakt die Negativform zum fleischfarbenen Material. Dies unterstützt die Festigkeit und Widerstandsfähigkeit der Verbindung, insbesondere einer Klebefuge, zwischen den beiden Materialien gegen darauf wirkende Kräfte, beispielsweise bei der weiteren Verarbeitung des Rohlings, aber auch bei der alltäglichen Benutzung der finalen Teilprothese.

In einer bevorzugten Ausgestaltung sind die Abstände zwischen den Erhöhungen und Vertiefungen und/oder deren Höhe beziehungsweise Tiefe nicht konstant über den gesamten Block oder Rohling. Z.B. können die Scheitellinien von Erhöhungen und Vertiefungen mit großen Abständen und/oder Höhen bzw. Tiefen durch den oder nahe beim Mittelpunkt des Blocks oder Rohlings verlaufen. Die Scheitellinien von Erhöhungen und Vertiefungen mit kleinen Abständen und/oder Höhen bzw. Tiefen verlaufen in dieser Ausgestaltung im Randbereich des Blocks oder Rohlings. Teilprothesen, die im Bereich der äußeren Linien herausgearbeitet, insbesondere gefräst werden, weisen bei dieser Ausführungsform automatisch kleinere Dimensionen auf als Teilprothesen, welche im Bereich der Linien, welche durch den oder nahe beim Mittelpunkt des Blocks oder Rohlings verlaufen, herausgearbeitet, insbesondere gefräst werden. Auch eine umgekehrte Gestaltung, also mit den großen Abständen und Höhen bzw. Tiefen an den beiden Randbereichen des Blocks oder Rohlings, ist möglich.

Auch können die Abstände zwischen den Erhöhungen und Vertiefungen und/oder deren Höhe beziehungsweise Tiefe graduell von einer Seite des Blocks oder Rohlings zur anderen hin zunehmen, oder in einer beliebigen anderen Anordnung ausgestaltet sein. Typischerweise werden häufiger Molaren und Prämolaren als Eckzähne und Schneidezähne benötigt und so kann durch die erfindungsgemäße Gestaltung der Platz im Rohling auf eine einfache Weise an die Anforderungen angepasst werden.

In einer weiteren erfindungsgemäßen Ausgestaltung ist es vorgesehen, das zahnfarbene Material mit einem Farbverlauf zu gestalten. Hierbei ist es vorgesehen, dass an der Grenzfläche zwischen den beiden Materialien ein dunklerer Farbton für das zahnfarbene Material gewählt wird und sich der Farbton so ändert, dass er sich mit zunehmendem Abstand zum fleischfarben Material aufhellt. Dies erlaubt es auf eine einfache Weise, durch die erfindungsgemäße Gestaltung des Blocks oder Rohlings eine naturgetreue Anmutung der Zähne im finalen Produkt zu erreichen, ohne dass eine aufwendige Nachbearbeitung erforderlich wäre, wie beispielsweise ein Bemalen der Zähne, um diese an die angrenzenden natürlichen Zähne des Patienten anzupassen.

Erfindungsgemäß kann aus einem Rohling eine Vielzahl an Teilprothesen, auch für unterschiedliche Patienten, präzise und ohne die Verwendung von (Wachs-)Modellen hergestellt werden. Dies gewährleistet somit den Ausschluss von Transferfehlern und gleichzeitig optimale Materialeigenschaften.

In der einteiligen Ausgestaltung ist durch den zweifarbig gestalteten Rohling ein Zusammenfügen zwischen Gingivabereich und Zahnanteil nicht mehr nötig.

Durch die erfindungsgemäß bevorzugte Ausgestaltung, nämlich durch die intensive Verbindung des zahnfarbenen und des fleischfarbenen Materials des Rohlings ergibt sich der Vorteil, dass die Fräsarbeiten wesentlich schneller vonstatten gehen können. Zum Bearbeitungsvorgang beider Materialien ist lediglich ein Einspannen erforderlich. Aufgrund der intensiven Verbindung zwischen dem zahnfarbenen und dem fleischfarbenen Material und aufgrund des Formschlusses zwischen diesen, reicht die Festigkeit der Verbindung überraschend auch für die Fräsbearbeitung und die dort eingebrachten Kräfte aus.

Besonders günstig ist es, dass die einteilige Prothese durch einen mechanischen Bearbeitungsvorgang, insbesondere durch Fräsen in einer CAD/CAM-Einheit, unter Einbindung einer Steuervorrichtung automatisch fertiggestellt werden kann. Mit dieser Steuervorrichtung lässt sich die genaue Lage der Teilprothese im Rohling automatisch und/oder benutzergesteuert festlegen.

Eine CAD/CAM-Vorrichtung stellt die dentale Teilprothese fertig, indem sie die genaue Platzierung der Teilprothese im Rohling basierend auf patientenspezifischen Daten, insbesondere patientenspezifischen Zahngrößen und -breiten, festlegt und basierend hierauf die Prothese erzeugt. Damit wird die Größe der Teilprothese patientenspezifisch festgelegt, und es kann auch unterschiedlichen Formen des jeweiligen Zahnbogenteils Rechnung getragen werden, also insofern unterschiedlicher Zahnverläufe. Die Bereitstellung der erforderlichen Patientendaten erfolgt folgendermaßen: Zunächst wird mittels eines herkömmlichen Intraoralscans oder einer Abdrucknahme mit anschließendem 3D-Scan dieser die Zahnsituation des Patienten aufgenommen und der Steuervorrichtung zugeleitet. Diese markiert anatomisch relevante Punkte beziehungsweise anatomische Punkte im relevanten Bereich für die spätere Teilprothese. Diese Punkte dienen als Referenzpunkte. Weiterhin legt die Steuervorrichtung basierend auf der von einer Scanvorrichtung erfassten Mundsituation eines Patienten individuelle Zahnformen, die Rotation und/oder die Angulation der Zähne und die Form der Basis der Teilprothese fest.

Diese Daten werden der Software der CAD/CAM-Vorrichtung zugeleitet. Zunächst werden hierzu die Daten der Ober- und Unterkiefermodelle importiert und lagerichtig zueinander positioniert. Anschließend erstellt die CAD/CAM-Vorrichtung automatisch mit ihrer Steuervorrichtung einen Vorschlag für eine Teilprothese, eine sogenannte virtuelle Teilprothese und gibt einen Vorschlag ab, an welcher Stelle des Rohlings diese optimalerweise hergestellt werden könnte. Die virtuelle Teilprothese und auch die vorgeschlagene Platzierung sind aber vom Benutzer, also z.B. vom Zahntechniker, an der CAD/CAM-Vorrichtung abänderbar.

In einer bevorzugten Ausgestaltung ist jeder Rohling mit einem individuellen Identifizierungsmerkmal, wie beispielsweise einem QR-Code, versehen und weist weiterhin Referenzpunkte auf, welche eine exakte Positionierung in der CAD/CAM-Vorrichtung erlauben. Alternativ ist auch die Realisierung mittels eines RFID-Chips hierzu möglich. Dies gilt überraschend auch, wenn der Rohling aus der Vorrichtung entfernt und später erneut eingespannt wird. Nachdem eine Teilprothese aus dem Rohling hergestellt wurde, speichert sich die Steuervorrichtung die exakte Position der bearbeiteten Stelle des Rohlings mit Hilfe der Referenzpunkte am Rohling sowie dem individuellen Identifizierungsmerkmal des Rohlings. Somit weiß die Steuervorrichtung automatisch, auf welchem Rohling welcher Bereich noch unbenutzt ist und schlägt für eine virtuelle Teilprothese automatisch einen passenden Rohling sowie eine optimale Platzierung auf diesem vor.

Es ist auch möglich, die CAM-Daten von bereits gefertigten Rohlingen für das Nesting zu verwenden. Ferner ist es möglich, eine Kamera in der Fräsmaschine vorzusehen, die auf den zu bearbeitenden, ggf. teilgefrästen, Rohling gerichtet ist und per Bilderkennung Informationen über die zur Verfügung stehenden Bereiche des Rohlings liefert.

Auch ist es möglich, dass die Steuervorrichtung ein sogenanntes Nesting mehrerer virtueller Teilprothesen auf einem oder mehreren Rohlingen vornimmt, so dass eine besonders gute Materialausnutzung erreicht wird. Hierzu nimmt die Steuervorrichtung eine Positionsoptimierung für die jeweiligen virtuellen Teilprothesen vor und berücksichtigt hierbei insbesondere auch schon teilbearbeitete Rohlinge. Ist eine optimale Platzierung nicht möglich, oder liegen nicht mehr ausreichend neue Rohlinge vor, gibt die Software ein entsprechendes Warnsignal ab.

Hat die Steuervorrichtung eine optimale Platzierung ermittelt, schlägt sie diese dem Benutzer, beispielsweise mittels eines Pop-Up Fensters, vor, so dass dieser diese bestätigen kann. Es ist aber auch möglich, die Platzierung automatisch an die CAD/CAM-Vorrichtung weiter zu geben, ohne eine Bestätigung des Benutzers zu benötigen. Hierbei werden die ermittelten Daten automatisch zur Weiterbearbeitung freigegeben und für die Erzeugung der Teilprothese einer Fräsmaschine der CAD/CAM-Vorrichtung zugeleitet, in die ein zweifarbiger erfindungsgemäßer Rohling, insbesondere ein zweifarbiger, bereits teilbearbeiteter Rohling, eingespannt ist oder wird und die erwünschte Teilprothese herstellt. Nach einer abschließenden Politur kann die fertige Teilprothese an den Zahnarzt zur Eingliederung geliefert werden.

In einer modifizierten Ausgestaltung ist es vorgesehen, dass beim Fräsvorgang Verbindungs-Stege zwischen der herzustellenden Teilprothese und dem restlichen Rohling verbleiben, so dass ein Herausfallen der Teilprothese während des Fräsvorgangs vermieden werden kann, auch wenn der Rohling im dreidimensionalen Raum rotiert oder gekippt werden sollte. Dies erlaubt es, beliebige herkömmliche Fräsmaschinen für einen erfindungsgemäßen Rohling zu verwenden, ohne dass spezielle Umbauten der Fräsmaschine erforderlich wären.

Durch die Steuervorrichtung lässt sich basierend auf den gewonnenen Patientendaten die individuelle Zahnform, aber auch die Rotation und die Angulation der Zähne festlegen, und ebenso die Form der Basis der Teilprothese, so dass Teilprothesen für jede in der Praxis vorkommende Zahnsituation eines Patienten realisierbar sind.

Somit sind für viele klinische Situationen, wie beispielsweise unterschiedliche Schaltlückenlängen, unterschiedliche Zahngrößen, unterschiedliche Zahnsegmente und/oder Teilprothesen individuell und monolithisch aus einem Rohling-Block oder einer Rohling-Scheibe fertigbar, so dass mit diesen Rohlingen sowie einem automatisierten Fertigungsverfahren der Herstellungsprozess von Teilprothesen deutlich vereinfacht werden kann.

Auch können verschieden große Rohlinge, also Rohlinge mit unterschiedlichen Gesamt-Größen, vergleichbar mit Konfektionsgrößen wie beispielsweise "L", "M" oder "S", realisiert werden.

Besonders vorteilhaft ist es, dass durch die erfindungsgemäße Gestaltung und die im Querschnitt abwechselnden Erhöhungen und Vertiefungen bei der fertig gestellten Teilprothese jede Erhöhung des zahnfarbenen Materials der Sichtkante des Zahns an seinem Zahnhals gegenüber der von dem fleischfarbenen Material gebildeten Gingiva folgt. Die Ähnlichkeit zu natürlichen Zähnen und Zahnfleisch ergibt sich überraschend einfach, indem das fleischfarbene Material, insbesondere durch Fräsen, mindestens teilweise so weit abgetragen ist, dass an der Vestibulärseite die Trennlinie zwischen dem ballig gefrästen zahnfarbenen und fleischfarbenen Materialien als Gingivalinie zurückspringt, und zwar insbesondere sowohl gegenüber dem fleischfarbenen als auch den zahnfarbenen Material im Übrigen. Somit ist die fertiggestellte Teilprothese kaum von den umliegenden Zähnen zu unterscheiden.

Durch die erfindungsgemäße Gestaltung der Grenzfläche zwischen dem zahnfarbenen und dem fleischfarbenen Material lassen sich mehrere Teilprothesen unterschiedlichster (Zahn-)Größen aus einem Rohling herstellen.

Erfindungsgemäß ist es besonders günstig, dass bei diesem Herstellungsprozess die einzelnen Zähne einer Teilprothese über Verbindungsstellen aus zahnfarbenem Material miteinander verbunden bleiben. Hierdurch ist es, im Vergleich zu herkömmlichen Teilprothesen, bei denen konventionelle Einzelzähne in eine Basis eingeklebt werden, überraschend möglich, die Widerstandsfähigkeit gegenüber Kaukräften, also insbesondere gegenüber seitlichen Scherkräften, zu verbessern.

Während einzeln gefertigte und konfektionierte Zähne typischerweise in Zahnkavitäten in einer gemäß dem Stand der Technik gefertigten Prothesenbasis aufgenommen sind und aufgrund der Hebelwirkung der Kaukräfte bei der Mastikation erheblichen Scherbelastungen unterworfen sind, die auch die Klebefläche stark belasten, ist es erfindungsgemäß vorgesehen, diese Scherkräfte über die erfindungsgemäße Gestaltung der Teilprothese, insbesondere der besonderen Gestaltung der Grenzfläche zwischen zahnfarbenem und fleischfarbenem Material, und der Verbindung der Zähne zu einer sich über die ganze Teilprothese erstreckende Brücke, zu minimieren. Erfindungsgemäß ist die gefürchtete Lockerung der Klebung des Einzelzahns eliminiert, denn die Verbindung von mindestens zwei benachbarten Zähne der Teilprothesen zeigt aufgrund der vergrößerten Verbundfläche bei der Mastikation geringere Scherbelastungen.

Weitere Vorteile, Einzelheiten und Merkmale ergeben sich aus der nachfolgenden Beschreibung mehrerer Ausführungsbeispiele der Erfindung anhand der Zeichnungen.

Es zeigen:
Fig. 1 einen schematischen Schnitt durch einen erfindungsgemäßen Block in perspektivischer Ansicht in möglichen Ausführungsformen;
Fig. 1a) einen schematischen Schnitt durch einen erfindungsgemäßen Block in perspektivischer Ansicht in einer ersten Ausführungsform mit möglichen Platzierungen verschiedener virtueller Teilprothesen;
Fig 1b) einen schematischen Schnitt durch einen erfindungsgemäßen Block in perspektivischer Ansicht in einer weiteren Ausführungsform;
Fig. 2 schematische Darstellungen möglicher Ausführungsformen eines erfindungsgemäßen Seitenzahn-Rohlings in Aufsicht und mögliche Positionierungen von Teilprothesen unterschiedlicher Größe;
Fig. 2a) schematische Darstellungen einer weiteren Ausführungsform der erfinderischen Grenzfläche zwischen dem zahnfarbenen und dem fleischfarbenen Material und mögliche Positionierungen von Zähnen unterschiedlicher Größe in einem scheibenförmigen Rohling;
Fig. 2b) schematische Darstellungen einer ersten Ausführungsform eines erfindungsgemäßen Blocks in Aufsicht und mögliche Positionierung einer Teilprothese;
Fig. 3 schematische Darstellungen möglicher Ausführungsformen eines erfindungsgemäßen Seitenzahn-Rohlings in Aufsicht und mögliche Positionierungen von Teilprothesen unterschiedlicher Größe;
Fig. 3a) schematische Darstellungen einer weiteren Ausführungsform der erfinderischen Grenzfläche zwischen dem zahnfarbenen und dem fleischfarbenen Material und mögliche Positionierungen von Zähnen unterschiedlicher Größe in einem scheibenförmigen Rohling;
Fig. 3b) schematische Darstellungen einer ersten Ausführungsform eines erfindungsgemäßen Blocks in Aufsicht und mögliche Positionierung einer Teilprothese;
Fig. 4 schematische Darstellungen möglicher Ausführungsformen eines erfindungsgemäßen scheibenförmigen Rohlings und Blocks in Aufsicht und mögliche Positionierungen von Teilprothesen unterschiedlicher Größe;
Fig. 4a) schematische Darstellungen einer weiteren Ausführungsform der erfinderischen Grenzfläche zwischen dem zahnfarbenen und dem fleischfarbenen Material und mögliche Positionierungen von Zähnen unterschiedlicher Größe in einem scheibenförmigen Rohling; und
Fig. 4b) schematische Darstellungen einer ersten Ausführungsform eines erfindungsgemäßen Blocks in Aufsicht und mögliche Positionierung einer Teilprothese;
Fig. 5 einen schematischen Schnitt durch einen erfindungsgemäß hergestellten Zahn-/Gingiva-Teil, bestehend aus einem zahnfleischfarbenen und einem zahnfarbenen Material in einer ersten Ausführungsform;
Fig. 5a) einen schematischen Schnitt durch einen erfindungsgemäß hergestellten Zahn-/Gingiva-Teil, bestehend aus einem zahnfleischfarbenen und einem zahnfarbenen Material in einer ersten Ausführungsform; und
Fig. 5b) einen schematischen Schnitt durch einen erfindungsgemäß hergestellten Teilprothesenanteil respektive Zahn-/Gingiva-Teil, bestehend aus einem zahnfleischfarbenen und einem zahnfarbenen Material in einer weiteren Ausführungsform.

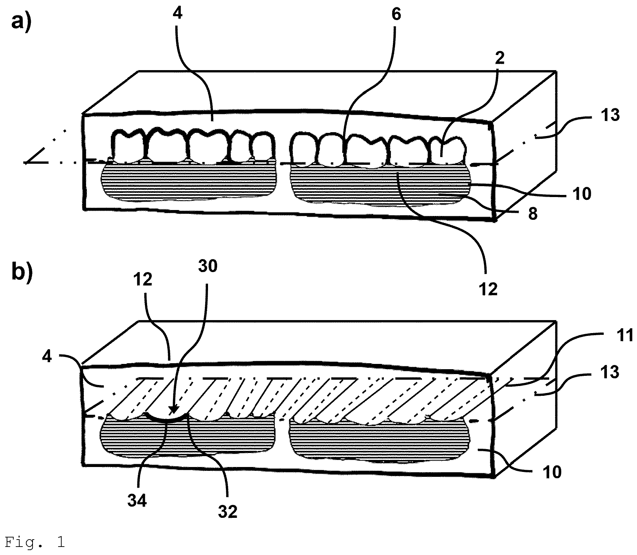

Fig. 1a) und b) zeigen einen schematischen Schnitt durch einen erfindungsgemäßen Block oder Rohling in perspektivischer Ansicht. In Fig. 1a) sind mögliche Platzierungen mehrerer virtueller Zahn-/Gingiva-Teile 1 eingezeichnet. Die, insbesondere durch Verbindungsstellen 6 verbundenen, Zähne 2 bestehen aus zahnfarbenem Material 4 und die Prothesenbasis 8 aus fleischfarbenem Material 10. Die beiden Materialien sind an deren Grenzfläche 12 intensiv miteinander verbunden, insbesondere aneinander anpolymerisiert oder verklebt.

Die Zahn-/Gingiva-Teile 1 werden aus einem zweifarbigen Block oder Rohling hergestellt. Die die Grenzfläche 12 durchtretende Ebene 13 zwischen den beiden Materialien verläuft senkrecht zur Höhe, und damit in den Fig. 1a) und 1b) horizontal. In Fig. 1a) ist die Ebene 13 in der Seitenansicht dargestellt.

Fig. 1b) zeigt die Anordnung der Grenzfläche 12 in einem erfindungsgemäßen Block mit den erfindungsgemäßen Scheitellinien 11 in perspektivischer Ansicht. Hierbei ist die dreidimensionale Ausgestaltung des fleischfarbenen Materials 10 an der Grenzfläche zwischen den Materialien 4 und 10 dargestellt.

In der Aufsicht verläuft die Ebene 13 als Schnittebene durch die erfindungsgemäße Grenzfläche 12 zwischen dem zahnfarbenen Material 4 und dem fleischfarben Material 10. Auch wenn dies in Fig. 1 so dargestellt ist: Die Ebene 13 und damit der generelle Verlauf die Grenzfläche 12 zwischen den beiden Materialien muss nicht horizontal im Rohling verlaufen. Vielmehr kann die Ebene 13 auch schräg oder auch gebogen verlaufen. Der Rohling kann blockförmig oder rund ausgestaltet sein. Bei der blockförmigen Ausgestaltung verläuft die die Grenzfläche 12 durchtretende Ebene 13 zwischen den beiden Materialien horizontal im flach liegenden Block. In einer anderen Ausgestaltung ist eine schräg im Rohling verlaufende, die Grenzfläche 12 durchtretende Ebene 13 und somit auch eine schräg im Rohling verlaufende Grenzfläche 12 realisiert.

Wie in Fig. 1b) dargestellt weist die Grenzfläche 12 zwischen den Materialien des Blocks, in der Ansicht entlang der Ebene 13 und senkrecht auf die erfindungsgemäßen Scheitellinien 11, eine Abfolge von Erhöhungen 32 und Vertiefungen 34 auf. Die Scheitellinien 11 einer Vertiefung 34 oder einer Erhöhung 32 bildet jeweils eine Linie, insbesondere eine gerade Linie, welche parallel oder zumindest annährend parallel zur Grenzfläche verläuft. Es ist aber auch eine leichte Neigung zur Grenzfläche hin oder von dieser weg möglich. Diese Neigung kann 0,5 Grad, 1 Grad, 2, Grad, 4 Grad, 7 Grad, 10 Grad, 15 Grad, 20 Grad oder einen beliebigen Wert zwischen 0 Grad und 30 Grad annehmen.

Weiterhin verlaufen die einzelnen Scheitellinien 11 der Erhöhungen 32 und Vertiefungen 34 jeweils im Wesentlichen parallel zueinander. Die Scheitellinien 11 ragen je aus der Ebene heraus. Die Scheitellinien 11 der Erhöhungen 32 erstrecken sich - bezogen auf die Figuren - oberhalb der Ebene 13 und die Scheitellinien 11 der Vertiefungen 34 unterhalb der Ebene 13.

Das zahnfarbene Material 4 weist an der Grenzfläche 12 zwischen den beiden Materialien exakt die Negativform zum fleischfarbenen Material 10 auf. Somit weist die Grenzfläche 12 zwischen den Materialien linienförmige Erhöhungen 32 und Vertiefungen 34 auf und die beiden Materialien ineinander greifen. Zwischen diesen erstrecken sich Übergänge, die Schrägflächen oder gleichsam Hänge bilden. Im Querschnitt, welcher senkrecht zu den linienförmigen Erhöhungen 32 und Vertiefungen 34 und auch senkrecht zur Ebene 13 verläuft, hat insofern die Grenzfläche 12 zwischen den beiden Materialien einen geschwungenen Verlauf. Diese Form kann auch als kettenlinienförmig bezeichnet werden.

Betrachtet man den Rohling wie in Fig. 1b) dargestellt im Querschnitt durch beide Materialien, also senkrecht zur Ebene der Grenzfläche 12 - wobei hier angenommen wird, dass das fleischfarbene Material 10 unten und das zahnfarbene Material 4 oben ist und welcher Querschnitt senkrecht zu den Scheitellinien 11 verläuft - sind die Erhöhungen 32 und Vertiefungen 34 des fleischfarbenen Materials 10 an der Grenzfläche 12 bei einer Spiegelung an der Ebene 13 nicht symmetrisch. Die Erhöhungen 32 sind vielmehr eher spitz zulaufend und die Vertiefungen 34 eher ausgerundet. Diese Gestaltung der Grenzfläche 12 entspricht dem menschlichen Zahnfleischsaum und kann mit einer Aneinanderreihung von Us 30 verglichen werden.

Das Ineinandergreifen unterstützt die Festigkeit und Widerstandsfähigkeit der Verbindung des zahnfarbenen Materials 4 mit dem fleischfarben Material 10, insbesondere bei einer Verbindung über eine Klebefuge, gegen darauf wirkende Kräfte, beispielsweise bei der weiteren Verarbeitung des Rohlings, aber auch bei der alltäglichen Benutzung der finalen Teilprothese. Die Asymmetrie der Grenzfläche hingegen erlaubt eine einfache Herstellung von Zahn-/Gingiva-Teilen 1, welche besonders natürlich wirken, da die spitz zulaufenden Erhöhungen 32 des fleischfarbenen Materials 10 automatisch dem natürlichen Verlauf des Zahnfleischs im Zwischenzahnbereich gleichen. Die ausgerundeten Vertiefungen 34 des fleischfarbenen Materials 10, also in der Negativform die ausgerundeten Erhöhungen des zahnfarbenen Materials 4, geben die natürliche Zahnform vor. Um eine natürliche Anmutung zu erreichen, müssen die Zahn-/Gingiva-Teile 1 somit nur an passenden Stellen, also an Stellen, die von der Steuervorrichtung der CAD/CAM-Vorrichtung als geeignet - und bevorzugt auch am platzsparendsten - ausgemacht werden, aus dem Rohling gefräst werden. Als finale Nachbearbeitung ist nur ein Schlichtfräsen des Zahnfleischsaums am Übergangsbereich zwischen fleischfarbenem und zahnfarbenem Material 4, 10 nötig.

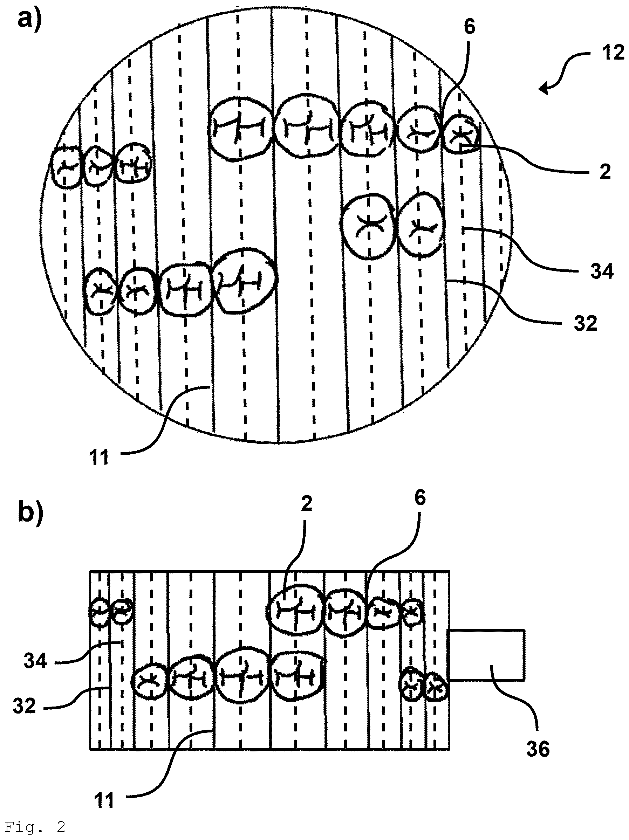

Die Fig. 2a) und 2b), 3a) und 3b) sowie 4a) und 4b) zeigen schematische Darstellungen mehrerer Ausführungsformen eines erfindungsgemäßen Rohlings, nämlich einer Scheibe in den Fig. 2a), 3a) und 4a) und einem Block in den Figuren 2b), 3b) und 4b). Die Darstellung in diesen Figuren soll je die Grenzfläche 12 zwischen den Materialien 4 und 10 (vgl. Fig. 1a) und b)) zeigen. Es sind je mehrere parallele Linien dargestellt, die je quer über die Scheibe bzw. den Block verlaufen. Gestrichelte Linien entsprechen hierbei Scheiteln oder Scheitellinien 11 der Vertiefungen 34 - diese Stelle entspricht dann je der Mitte eines Zahns der hergestellten Teilprothese. Durchgezogene Linien entsprechen Scheiteln oder Scheitellinien 11 der Erhöhungen 32, die den Zahnzwischenräumen, den Papillen, also in der Teilprothese den Verbindungsstellen 6 zwischen den einzelnen Zähnen 2, entsprechen. Beide Angaben sind auf das fleischfarbene Material 10 bezogen.

Ferner sind jeweils mögliche Positionen von Zähnen und Zahnanordungen 2 in Fig. 2a) und 2b) eingezeichnet. Diese entsprechen den Zähnen der Teilprothesen, die aus dem Block gemäß Fig. 2b) und der Scheibe gemäß Fig. 2a) später herausgefräst werden sollen.

Ein weiterer besonderer Vorteil eines z.B. einen Durchmesser von knapp 100 mm aufweisenden scheibenförmigen Rohlings liegt darin begründet, dass eine Vielzahl von Positionen für die Anordnung von Teilprothesen zur Verfügung steht. Das solches Rohling lässt sich auch für erheblich mehr Teilprothese verwenden als in Fig. 2a) dargestellt. Die sich vollständig und ununterbrochen quer über den Rohling erstreckenden Scheitellinien der Erhöhungen und Vertiefungen ermöglichen beliebig verteilte Anordnungen der Teilprothesen, auch, wie in Fig. 2a) darstellt, angrenzend an der Rand des scheibenförmigen Rohlings.

Fig. 2a) und 2b) zeigt eine Wellenform in der Grenzfläche 12 zwischen den Materialien 4 und 10, bei der die Abstände zwischen den Scheitellinien 11, welche nahe des Zentrums des Rohlings liegen, weiter auseinander liegen als die Abstände zwischen Scheitellinien 11 näher am Rand des Rohlings. Dies führt dazu, dass hier nahe des Zentrums des Rohlings große Zahngrößen und nahe am Rande des Rohlings kleinere Zahngrößen realisiert werden können. Eine geringerer Linienabstand entspricht einer weniger hohen Wellenform und ein größerer einer höheren Wellenform.

Wenn die Zähne einer Teilprothese oder eines Zahn-/Gingiva-Teils also groß sein sollen, positioniert man die Teilprothese in die Mitte, und wenn klein, an den linken oder rechten Rand des Rohlings.

Es versteht sich, dass zudem die genaue Form der Kettenlinie oder Welle, also gleichsam die Form der Abhänge zwischen dem Erhöhungen und Vertiefungen, nach Belieben variiert werden kann, so dass bei Bedarf massigere oder schlankere Zähne realisierbar sind.

Es ist auch möglich, die Form dieser Schrägflächen oder Abhänge entlang der Scheitellinien 11 zu variieren. Die Wahl der Position der Zähne auf den Scheitellinien 11 entscheidet dann über die Formgebung des betreffenden Einzelzahns oder der Zähne der Teilprothese.

Auch wenn in den Fig. 2 bis 4 lediglich Prämolare und Molare, die die Teilprothese bilden, dargestellt sind, versteht es sich, dass gleichermaßen auch Teilprothesen, die Schneide- und/oder Eckzähne umfassen, erzeugt werden können, in einem dem menschlichen Zahnbogen entsprechenden, gekrümmten Verlauf der Zahnanordnung.

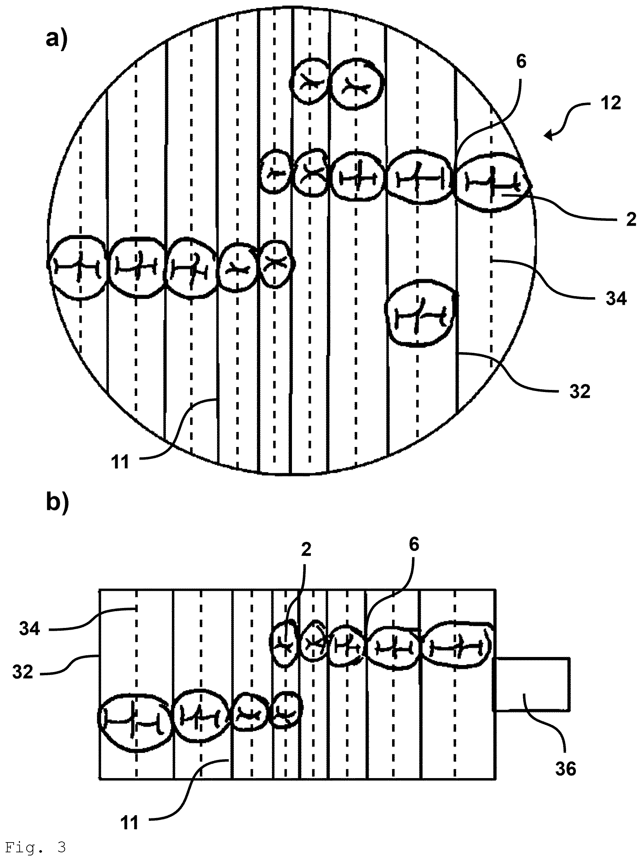

Die Ausführungsform gemäß Fig. 3a) und b) unterscheidet sich von der gemäß Fig. 2a) und 2b) lediglich durch die Linienverteilung. Hier sind die Abstände zwischen den Scheitellinien 11, welche nahe des Zentrums des Rohlings liegen, näher zusammen als die Abstände zwischen Scheitellinien 11 näher am Rand des Rohlings. Bei dieser Ausführungsform sind kleine Zahngrößen im mittleren Bereich des Rohlings und große Zahngrößen am linken oder rechten Rand des Rohlings vorgesehen.

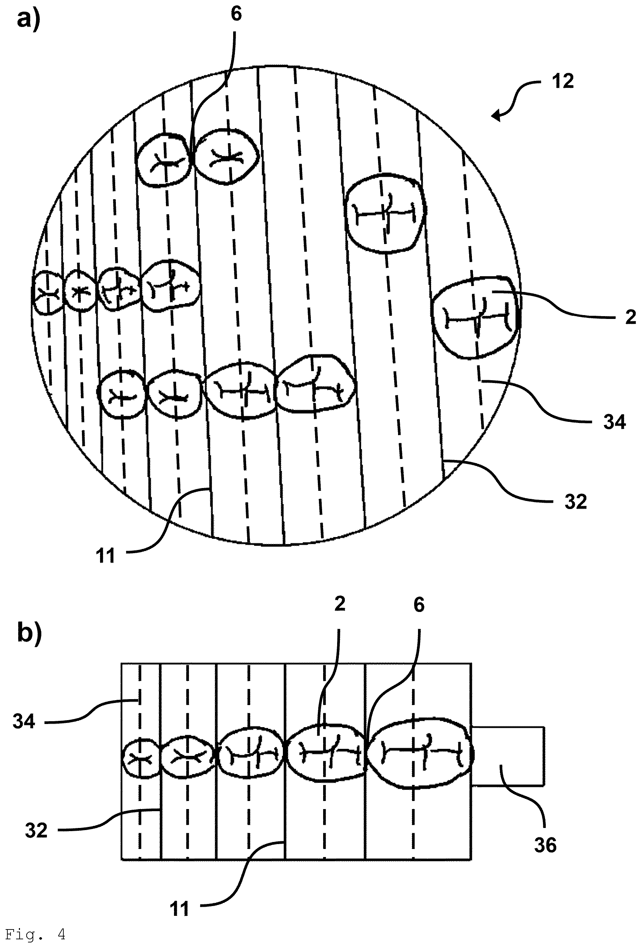

Die Ausführungsform gemäß Fig. 4a) und b) unterscheidet sich von der gemäß Fig. 2a) und 2b) sowie 3a) und 3b) ebenfalls durch die Linienverteilung. Hier sind die Abstände zwischen den Scheitellinien 11 weiter links im Rohling geringer als die Abstände zwischen Scheitellinien 11 weiter rechts. Dementsprechend können kleine Zahngrößen im linken Bereich des Rohlings und große Zahngrößen im rechten Bereich des Rohlings angeordnet werden.

Anstelle der linearen Zu- bzw. Abnahme des Abstandes der Scheitellinien 11 - wie hier dargestellt - ist auch eine logarithmische oder auch eine unregelmäßige bzw. beliebige Zu-/Abnahme der Linienabstände möglich, um besonderen Anforderungen gerecht zu werden.

Ferner ist es auch möglich, die Scheitellinienschar über Ihren Verlauf divergieren zu lassen. Die Abweichung von der Parallelität kann bei benachbarten Scheitellinien 0,5 Grad, 1 Grad, 2, Grad, 4 Grad, 7 Grad, 10 Grad, 15 Grad, 20 Grad oder einen beliebigen Wert zwischen 0 Grad und 30 Grad annehmen.

Auch ist es nicht erforderlich, dass die Scheitellinien 11 exakt gerade verlaufen. Der Verlauf kann eine Wellung oder sonstige Richtungsänderung aufweisen, und zwar sowohl in Richtung der benachbarten Scheitellinien 11 als auch in Höhenrichtung. Dies erlaubt auf eine einfache Weise Größenvariationen der herzustellenden Zahn-/Gingiva-Teile 1 und insbesondere unregelmässige Abweichungen der Zahngrößen, also beispielsweise einen ungewöhnlich kleinen Zahn zwischen normal großen Zähnen, oder umgekehrt. Eine derartige Formabfolge findet sich manchmal auch bei natürlichen Zähnen.

Die erfindungsgemäßen Blöcke können in verschiedenen Größen gefertigt werden, um eine feinere Anpassbarkeit der Dimensionierung zu erreichen. Blöcke in den Größen "L" können für große Zahn-/Gingiva-Teile, "M" für durchschnittlich große Zahn-/Gingiva-Teile und "S" für kleine Zahn-/Gingiva-Teile hergestellt werden.

In den Fig. 2b), 3b) und 4b) ist ein Halter 36 dargestellt, welcher zur Einspannung des erfindungsgemäßen Blocks in der Einspann-Vorrichtung der CAM-Vorrichtung dient. Dieser Halter 36 kann beispielsweise an den erfindungsgemäßen Block angeklebt sein.

Auch ist eine Schraubverbindung des Halters 36 am Block, eine formschlüssige Steckverbindung oder eine einstückige Ausgestaltung des Halters 36 und des Blocks möglich. Der Halter 36 kann beispielsweise aus fleischfarbenem und/oder aus zahnfarbenem Material, aus einem anderen Kunststoff oder auch aus Metall sein und in seiner Form an die Erfordernisse der Einspannvorrichtung der jeweiligen CAM-Vorrichtung angepasst sein.

Es ist auch möglich, dass der Block mit einer gegenüber den dargestellten Ausführungsbeispielen um z.B. 45° gedrehten Struktur hergestellt wird. Die Scheitellinien 11 sind dann nicht parallel zu einer Seitenfläche des Blocks, sondern diagonal oder schräg und im Wesentlichen parallel zur Grenzfläche des Blocks.

Es versteht sich, dass es für die Herstellung eines quaderförmigen Blocks, wie in den Fig. 2b), 3b) und 4b) dargestellt, nicht erforderlich ist, diesen aus einem scheibenförmigen Rohling, wie in den Fig. 2a), 3a) und 4a) dargestellt, zu fräsen. Vielmehr kann der Block direkt in Quaderform hergestellt werden.

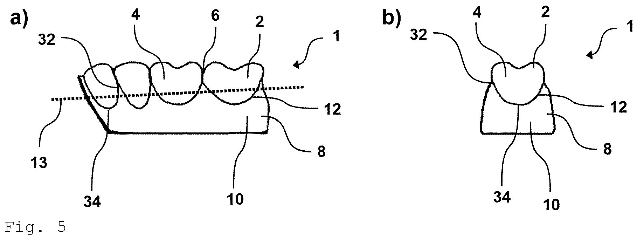

Fig. 5a) und b) zeigen einen schematischen Schnitt durch einen erfindungsgemäßen und aus dem Rohling oder Block gefrästen Zahn-/Gingiva-Teil 1. Die Zähne 2 bestehen aus zahnfarbenem Material 4 und der Gingiva-Teil 8 aus fleischfarbenem Material 10. Die beiden Materialien sind an deren Grenzfläche 12 vorab, also schon im Rohling, intensiv miteinander verbunden, insbesondere aneinander anpolymerisiert oder verklebt. Fig. 5a zeigt einen mehrteiligen, hier einen viergliedrigen, Zahn-/Gingiva-Teil. Die Zähne 2 aus zahnfarbenem Material 4 sind an Verbindungsstellen 6 miteinander verbunden.

Fig. 5b zeigt einen einteiligen Zahn-/Gingiva-Teil, eine sogenannte Einzelzahn-Prothese. Die Zahn-/Gingiva-Teile 1 werden aus einem flachen, ggf. scheibenförmigen, zweifarbigen Block oder Rohling, wie er in den Fig. 1 bis 4 dargestellt ist, hergestellt.

## Patentansprüche

1. Block mit einer oberen und einer unteren Fläche, wobei der Block aus einem fleischfarbenen Material und einem zahnfarbenen Material wobei das fleischfarbene Material und das zahnfarbene Material miteinander verbunden sind und wobei eine Grenzfläche zwischen den Materialien mit in oder an der Grenzfläche ausgebildeten Erhöhungen und Vertiefungen sich durch eine gegebenenfalls gebogene Ebene erstreckt, welche Ebene parallel zu oder schräg zu mindestens einem Teil der oberen und unteren Flächen des Rohlings liegt, **dadurch gekennzeichnet, dass** Scheitellinien der Erhöhungen und Vertiefungen, in der Draufsicht auf die Grenzfläche betrachtet, im Wesentlichen gerade Linien ausbilden und über den Block betrachtet fächerförmig angeordnet sind oder voneinander divergieren.

2. Block nach Anspruch 1, **dadurch gekennzeichnet, dass** die Scheitellinien der Erhöhungen und Vertiefungen parallel oder im Wesentlichen parallel zur Grenzfläche verlaufen.

3. Block nach einem der vorhergehenden Ansprüche, **dadurch gekennzeichnet, dass** die Scheitellinien einen konstanten Abstand zueinander aufweisen, oder, dass die Abstände zwischen Scheitellinien - in der Draufsicht auf die Grenzfläche betrachtet unterschiedlich sind, insbesondere graduell zu- und/oder abnehmen.

4. Block nach einem der vorhergehenden Ansprüche, **dadurch gekennzeichnet, dass** das zahnfarbene Material, und/oder ggf. das fleischfarbene Material mit einem Farbverlauf gestaltet ist und/oder sich mit zunehmendem Abstand von der Grenzfläche zwischen den Materialien, insbesondere kontinuierlich, zunehmend transparenter ist.

5. Dentaler Rohling mit einer oberen und einer unteren Fläche, wobei der Rohling aus einem fleischfarbenen Material und einem zahnfarbenen Material besteht, wobei das fleischfarbene Material und das zahnfarbene Material miteinander verbunden sind und wobei eine Grenzfläche zwischen den Materialien mit in oder an der Grenzfläche ausgebildeten Erhöhungen und Vertiefungen sich durch eine gegebenenfalls gebogene Ebene erstreckt, welche Ebene parallel zu oder schräg zu mindestens einem Teil der oberen und unteren Flächen des Rohlings liegt, **dadurch gekennzeichnet, dass** Scheitellinien der Erhöhungen und Vertiefungen, in der Draufsicht auf die Grenzfläche betrachtet, im Wesentlichen gerade Linien ausbilden und über den Block betrachtet fächerförmig angeordnet sind oder voneinander divergieren.

6. Dentaler Rohling nach Anspruch 5, **dadurch gekennzeichnet, dass** die Scheitellinien der Erhöhungen und Vertiefungen parallel oder im Wesentlichen parallel zur Grenzfläche verlaufen und/oder sich quer über den Rohling erstrecken.

7. Dentale Teilprothese, hergestellt aus einem Block gemäß einem der Ansprüche 1 bis 4 oder einem Rohling gemäß einem der Ansprüche 5 bis 6.

8. Verfahren zur Herstellung einer dentalen Teilprothese unter Verwendung einer CAD/CAM-Vorrichtung aus einem dentalen Rohling oder Block, der mit einer oberen und einer unteren Fläche gefertigt ist und aus einem fleischfarbenen Material und einem zahnfarbenen Material, aufgebaut wird, wobei das fleischfarbene Material und das zahnfarbene Material miteinander verbunden werden und wobei die Grenzfläche zwischen den Materialien mit in der Grenzfläche ausgebildeten Erhöhungen und Vertiefungen sich durch eine Ebene erstreckt, welche Ebene parallel oder gebogen oder schräg zu einer der Scheibenflächen des dentalen Rohlings oder Blocks liegt, **dadurch gekennzeichnet, dass** im dentalen Rohling oder Block ein Bereich für mindestens eine Teilprothese reserviert wird, der sich über eine oder mehrere Erhöhungen und Vertiefungen erstreckt, und dass Scheitellinien der Erhöhungen und Vertiefungen je im Wesentlichen gerade Linien ausbilden, und über den Block betrachtet fächerförmig angeordnet sind oder voneinander divergieren.

9. Verfahren zur Herstellung einer dentalen Teilprothese nach Anspruch 8, **dadurch gekennzeichnet, dass** die Scheitellinien der Erhöhungen und Vertiefungen parallel oder im Wesentlichen parallel zur Grenzfläche verlaufen.

10. Verfahren zur Herstellung einer dentalen Teilprothese nach einem der Ansprüche 8 oder 9, **dadurch gekennzeichnet, dass** aus einem dentalen Rohling oder Block eine Vielzahl von Teilprothesen, auch für unterschiedliche Patienten, insbesondere nach einem Scannen, einem virtuellen Erstellen der Prothese und virtueller Positionierung im Rohling, hergestellt, werden.

11. Verfahren zur Herstellung einer dentalen Teilprothese nach einem der Ansprüche 8 bis 10, **dadurch gekennzeichnet, dass** der Block Referenzpunkte für die Positionierung in der CAD/CAM-Vorrichtung und/oder ein individuelles Identifizierungskennzeichen, wie beispielsweise einen QR-Code oder ein RFID-Tag, aufweist.

12. Verfahren nach einem der Ansprüche 8 bis 11, **dadurch gekennzeichnet, dass** die CAD/CAM-Vorrichtung die Teilprothese hinsichtlich ihrer Raumform festlegt, und eine Steuervorrichtung aufweist, die in Abhängigkeit von der erwünschten Größe und/oder Zahnbreite die Teilprothese so in dem Rohling positioniert, und insbesondere auch die individuelle Zahnform, die Rotation und/oder die Angulation der Zähne vorschlägt und - ggf. nach Benutzereingriff - festlegt.

13. Verfahren nach einem der Ansprüche 8 bis 12, **dadurch gekennzeichnet, dass** die Steuervorrichtung sich für jeden Rohling die Position einer hergestellten Prothese speichert und für neue, herzustellende Teilprothesen, insbesondere durch ein "Nesting"-Verfahren, einen Rohling und/oder die Positionierung auf einem Rohling bestimmt, um eine maximale Rohstoffnutzung zu erreichen.
